# EUROPEAN PATENT SPECIFICATION

(11) **EP 3 897 556 B1**
(45) Date of publication and mention of the grant of the patent: **24.09.2025**
(21) Application number: 19831747.1
(22) Date of filing: 20.12.2019
(51) Int. Cl.: A61K 8/36, A61K 8/37, A61K 8/99, A61Q 19/00, A61Q 19/08

(54) **COSMETIC USE OF A SHORT CHAIN FATTY ACID (SCFA) FOR PREVENTING AND/OR TREATING DRY SKIN AND/OR AGED SKIN**
KOSMETISCHE VERWENDUNG EINER KURZKETTIGEN FETTSÄURE (SCFA) ZUR VORBEUGUNG UND/ODER BEHANDLUNG VON TROCKENER HAUT UND/ODER GEALTERTER HAUT
UTILISATION COSMÉTIQUE D'UN ACIDE GRAS À COURTE CHAÎNE (SCFA) POUR PRÉVENIR ET/OU TRAITER LA PEAU SÈCHE ET/OU LA PEAU ÂGÉE

(30) Priority: 21.12.2018 SG 10201811547R
(43) Date of publication of application: 27.10.2021
(73) Proprietor: L'OREAL, 75008 Paris (FR); Nanyang Technological University, Singapore 639798 (SG); The National University of Singapore, Singapore 119077 (SG)
(72) Inventor: RICE, Scott, Singapore Centre for Environmental L The School of Biological Sciences - Nany 60 Nanyang Drive, SBS-01N-27 Singapore 637551 (SG); BRILLET, François, 56270 PLOEMEUR (FR); CHOPRA, Tarun, Singapore 138623 (SG)
(74) Representative: Lavoix
(86) International application number: PCT/EP2019/086841
(87) International publication number: WO 2020/128071

(56) References cited:
- EP-A1- 1 508 328
- EP-A2- 0 508 324
- DE-A1- 19 529 773
- FR-A1- 2 996 452
- GB-A- 1 589 224
- US-A- 4 424 234
- US-A1- 2015 044 317
- US-B2- 9 782 611
- G TAX ET AL: "Propionic Acid Produced by Propionibacterium acnes Strains Contributes to Their Pathogenicity", ACTA DERMATO-VENEREOLOGICA., vol. 96, no. 1, 3 June 2015 (2015-06-03), United Kingdom, pages 43 - 49, XP055680339, ISSN: 0001-5555, DOI: 10.2340/00015555-2154

## Description

The present invention concerns the cosmetic field, and especially cosmetic uses for the prevention and/or treatment of dry skin and/or aged skin, such as hyposeborrheic dry skin and/or aged skin.

Skin is a complex matrix of tissues and mediates many functions for the human body. For this, the skin is equipped with specialized cells and mechanisms, many of which are unique to this organ.

Human skin consists of two compartments, namely a superficial compartment, the epidermis, and a deep compartment, the dermis. The natural human epidermis is composed mainly of four types of cells, which are: keratinocytes (make up the vast majority), melanocytes, sebocytes and Langerhans cells.

Each of these cell types contributes by its own functions to the essential role played in the body by the skin. In particular, sebocytes present in the sebaceous glands of the skin are cells that synthesize an oily substance called sebum (Schneider and Paus, 2010).

Sebum is a product excreted by the sebaceous glands of the skin of most mammals. The excretion mechanism is a holocrine mechanism by which sebum is deposited on the surface of the skin. One of the roles of sebum is to provide the skin with a hydrophobic coating through the sebaceous channels. In addition, human sebum has unique characteristics compared to other mammals. In particular, it contains a very small portion of cholesterol derivatives and a significant amount of squalene. Especially, sebum is a mixture of triglycerides, wax esters, squalene, cholesterol esters, cholesterol and free fatty acid (Pappas, 2009; Picardo et al., 2009).

The importance of sebum in the homeostasis of cutaneous tissue is known: sebum-deficient parts of the human body (soles of the feet and palms of the hands) reveal a morphology and aesthetics of skin surface that are profoundly different from that of the rest of the skin. Similarly, in the field of skincare, it appears that certain unsightly discomfort (dry skin) as well as significant dermatological disorders (premature aging) are attributed to the absence or insufficiency of sebum on the skin surface (Man et al., 2009; Yamamoto et al., 1987 ; Rogers et al., 1996; Seyfarth et al., 2011). An example of hyposeborrheic dry skin, or of skin becoming so, is observed during skin aging. Thus, the manifestation of xerosis related to a sebum deficiency is very commonly observed in elderly individuals, and in particular individuals over the age of 50 (Yamamoto, A., Serizawa, S., Ito, M. & Sato, Y. Effect of aging on sebaceous gland activity and on the fatty acid composition of wax esters. J. Invest. Dermatol. 89, 507-512 (1987)).

Thus, there is a need to provide novel active agents which can be useful for stimulating sebogenesis, and consequently for preventing and/or treating dry skin and/or aged skin, in particular hyposeborrheic dry skin and/or aged skin.

EP1 508 328 A1, DE 195 29 773 A1, EP 0 508 324 A2, GB 1 589 224 A and US 4 424 234 A disclose the use of specific short chain fatty acids or salts thereof (e.g. sodium butyrate, acid acetic, propionic acid) for treating and preventing dry or aged skin.

It is already known from US20090022819, that lysate from a filamentous bacteria. *Vitreoscilla filiformis* can be used for the prevention and/or the treatment of dry skin.

Moreover, the use of a *bifidobacterium* lysate for the prevention and/or the treatment of dry skin is described in US20090060962.

However, in these applications, the active agent consists of the biomass of bacteria. One of the disadvantages of the implementation of this biomass is that its incorporation into cosmetic compositions is likely to cause stabilization problems, and in particular a phase separation induced by sedimentation of the biomass.

The object of the present invention is, in particular, to satisfy these needs.

The present inventors surprisingly discovered that specific microbial metabolites called short chain fatty acids (SCFA) comprising from 3 to 8 carbon atoms, especially propionate, butyrate, and valerate, increase lipids sebum synthesis as well as sebum secretion, and consequently prevent and/or treat dry skin and/or aged skin, in particular associated with a lack of sebum secretion.

They further discovered that the use of long chain fatty acids (LCFA) comprising at least 10 carbon atoms in combination with short chain fatty acids (SCFA) boost the sebogenic response.

Propionic acid is known from CTFA (personal care products council) and from Shu, M. et al. [Fermentation of Propionibacterium acnes, a commensal bacterium in the human skin microbiome, as skin probiotics against methicillin-resistant Staphylococcus aureus. PloS One 8, e55380 (2013)] as an antimicrobial active agent and its salts (propionate) as pH corrector and have never been disclosed as acting on lipid production and sebum secretion.

Also, among SCFA known in cosmetics, acetate (a SCFA comprising 2 carbon atoms) has been disclosed as a lipid production inducer (Acne and Its Therapy, Guy F. Webster, Anthony V. Rawlings, p262). However, acetate only contributes to lipid production and not sebum secretion, as shown in the present application (see example 1, b1, Table 2). These two functions, which are lipid production and sebum secretion, are needed for an efficient treatment of a hyposeborrheic skin.

The present invention relates to the cosmetic use of a conditioned culture medium obtained from at least one microorganism of the species *Propionibacterium acnes,* the said medium comprising at least one short chain fatty acid comprising from 3 to 8 carbon atoms, salts thereof, esters thereof and mixtures thereof, for preventing and/or treating dry skin and/or aged skin, in particular hyposeborrheic dry skin and/or aged skin.

Another object of the present invention relates to the cosmetic method for preventing and/or treating dry skin and/or aged skin, in particular hyposeborrheic dry skin and/or aged skin, comprising the application of a cosmetic composition on the skin, wherein the said composition comprises a conditioned culture medium obtained from at least one microorganism of the species *Propionibacterium acnes,* the said medium comprising at least one short chain fatty acid comprising from 3 to 8 carbon atoms, salts thereof, esters thereof and mixtures thereof.

### Definitions

In the context of the invention, the term "skin" means any cutaneous surface of the body, preferentially the skin of the face and the scalp. It is intended to that the skin concerned in the present application is human skin.

As used herein, the term "treating" or "treatment" refers to any action that aims to improve the comfort or the well-being of an individual. This term therefore covers attenuating, relieving or suppressing the symptoms of dry skin and/or aged skin, but is limited to a cosmetic treatment.

For the purposes of the present invention, the term "preventing" means reducing the risk if manifestation of a phenomenon, especially in the context of the invention dry skin and/or aged skin.

The term "hyposeborrheic" is intended to mean a lack of sebum secretion, in particular an absence or a reduced secretion of sebum from the sebaceous glands. Conventionally, a sebum content of less than 100 µg/cm², measured at the T zone of the face, by the method described in FR2368708, can be considered to be characteristic of hyposeborrheic dry skin and aged skin.

For the purposes of the present invention, the terms "effective amount" means an amount that is sufficient to obtain the expected effect.

As used herein, the terms "cosmetic composition" means a composition suitable for an application on the skin, in particular a composition which comprises a physiologically acceptable medium.

The terms "physiologically acceptable medium" means a medium that is suitable for the topical administration of a composition, i.e. that is compatible (not toxic) with the skin of the face, the body and the scalp.

For the purposes of the present invention, the terms "short chain fatty acid" means a carboxylic acid with an aliphatic chain comprising 3 to 8 carbon atoms, preferably, a carboxylic acid with an aliphatic chain comprising 3 to 5 carbon atoms.

### Detailed description of the invention

### Short chain fatty acids (SCFA)

The short chain fatty acids according to the invention comprise a straight or branched, saturated or unsaturated aliphatic chain containing 3 to 8 carbon atoms, preferably 3 to 5 carbon atoms.

In a preferred embodiment, the short chain fatty acids according to the invention comprise a straight and saturated aliphatic chain containing 3 to 5 carbon atoms.

Among the short chain fatty acids, mention may be made particularly of propionic acid, butyric acid, valeric acid, and mixtures thereof.

Advantageously, the short chain fatty acids according to the invention does not contain a hydroxylated aliphatic chain, preferably, the short chain fatty acids are not alpha hydroxy acids and beta hydroxy acids, salts thereof, esters thereof, in particular are not lactic acid, salts thereof, esters thereof.

The salts of short chain fatty acids according to the invention are particularly preferred and may be any safe and effective salt of such acid. To illustrate, certain preferred salts may include calcium salts, sodium salts, magnesium salts, and potassium salts, the most particularly preferred being sodium salts.

As an additional illustration, amino acid salts may be utilized. For example, a carnitine or lysine salt of short chain fatty acids according to the invention may be utilized. The ordinarily skilled artisan will recognize that various other amino acids may be utilized as well.

As examples of esters of acetic acid, propionic acid, or butyric acid, any safe and effective ester of such acid may be utilized. For example, where the SCFA is an ester of propionic acid, the component can be represented as follows: 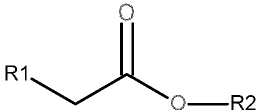 where R1 = CH₃ and R2 is the ester chain or the ester of propionic acid.

As an example, the ester chain of the selected acid may be a straight or branched chain of carbon atoms and typically contains about 8 carbon atoms or less. This ester chain more preferably contains from 1 to about 5 carbon atoms and, again, may be a straight (for example, n- propyl) or branched (for example, iso-propyl) chain. Highly preferred ester chains include those that form methyl esters (i. e., R2 is-CH3), ethyl esters, n-propyl esters, iso-propyl esters, n-butyl esters, iso-butyl esters, and mixtures thereof. To illustrate, methyl propionate, ethyl propionate, n-propyl propionate, iso-propyl propionate, n-butyl propionate, iso-butyl propionate are examples of esters of propionic acid that may be used herein. Such esters of propionic, butyric or valeric acid may be selected as well.

The short chain fatty acids according to the invention are contained in a conditioned culture medium (or supernatant) from at least one microorganism of the species *Propionibacterium acnes,* preferably from at least one microorganism of the strain *Propionibacterium acnes* ATCC 6919 as defined below.

As examples of short chain fatty acids, mention may be made of sodium propionate (Ref. P1880); Sodium butyrate (Ref. 303410); valeric acid (Ref. 75054) sold by Sigma.

The short chain fatty acids according to the present invention are used in an amount representing from 0,01% to 5% by weight relative to the total weight of the composition, preferably in an amount representing from 0,025% to 0,15% by weight relative to the total weight of the composition.

The present invention relates to the cosmetic use of a conditioned culture medium obtained from at least one microorganism of the species *Propionibacterium acnes,* the said medium comprising at least one short chain fatty acid comprising from 3 to 8 carbon atoms, salts thereof, esters thereof and mixtures thereof, for preventing and/or treating dry skin and/or aged skin, in particular hyposeborrheic dry skin and/or aged skin.

A "culture supernatant" also called "conditioned culture medium" is typically obtained by culturing the microorganism concerned in a medium suitable for survival and/or the growth of the microorganism, then by separation of the medium and the microorganism so as to harvest the medium brought into contact with the microorganism. Preferably, the culture is carried out for a period of time and under conditions likely to allow the microorganism to release in the medium the active agents having the seborrheic properties desired, in particular the short chain fatty acids (SCFA) according to the invention.

The environment suitable for the survival and/or growth of the microorganism will be any nutrient media suitable for survival and/or culture of the microorganism. It usually contains a source of carbon and nitrogen, such as, for example, amino acids, sugars, proteins, fatty acids, phosphates, sulphates, minerals and growth factors and vitamins in adequate amounts.

For the purposes of the present application, the terms "conditioned culture medium" or "culture supernatant" are used indifferently to designate the entirety of the culture supernatant obtained after culture of the microorganism in question, or any fraction or sub-compound of the supernatant obtained by dialysis, fractionation, phase separation, filtration chromatography, affinity chromatography, precipitation, concentration, lyophilization, etc.

In the context of the present invention, conditioned culture medium from at least one microorganism of the species *Propionibacterium acnes* according to the invention is obtained by the process comprising the following steps:
i) culturing at least one microorganism of the species *Propionibacterium acnes* such as *Propionibacterium acnes* ATCC 6919;
ii) separating, in particular by centrifugation, the culture supernatant from the biomass;
iii) recovering the culture supernatant; and
iv) optionally stabilizing the culture supernantant, for instance by filtration.

As used herein, the term "biomass" refers to the *Propionibacterium acnes* cells obtained after conducting the step i).

Preferably, the filtration is conducted with a syringe filter of pore size of 0,45µm.

### Long chain fatty acids

The short chain fatty acids according to the invention can be used in combination with at least one long chain fatty acid (LCFA) comprising at least 10 carbon atoms, non-glyceryl esters thereof, mixtures thereof.

As used herein, the LCFA contains a fatty acid chain, or wherein the fatty acid material is a fatty acid ester, contains a fatty acid chain and an ester chain.

Thus, wherein the LCFA is a fatty acid, the material can be represented as follows:

R-COOH

wherein "R" is the fatty acid chain which is a saturated or unsaturated chain having from about 10 to about 24 carbon atoms, and wherein "COOH" is a carboxylic acid moiety.

More preferably, "R" is a saturated or unsaturated chain having from about 12 to about 24, most preferably from about 16 to about 18 carbon atoms.

Also preferably, the fatty acid chain contains from 0 to about 3 double bonds.

Most preferably, the fatty acid chain is unsaturated, in particular having one or two double bonds.

Wherein the LCFA is a non-glyceryl ester of a fatty acid (i. e., a "non-glyceryl ester thereof"), the material can be represented as follows:

R-COOR'

wherein R is the fatty acid chain as defined above, and R' is the ester chain, with the carboxylate moiety "COO".

The ester chain is a straight or branched chain of carbon atoms and typically contains about 8 carbon atoms or less. The ester chain more preferably contains from 1 to about 5 carbon atoms and, again, may be a straight (for example, n- propyl) or branched (for example, iso-propyl) chain. Highly preferred ester chains include those that form methyl esters (i. e. , R' is-CH3), ethyl esters, n-propyl esters, iso-propyl esters, n-butyl esters, iso-butyl esters, and mixtures thereof. Those ester chains that form ethyl esters are particularly preferred.

In a preferred embodiment of the present invention, the LCFA is selected from lauric acid, lauroleic acid, myristic acid, myristoleic acid, pentadecanoic acid, palmitic acid, palmitoleic acid, margaric acid, stearic acid, dihydroxystearic acid, oleic acid, ricinoleic acid, elaidic acid, linoleic acid, alpha-linolenic acid, dihomogamma-linolenic acid, eleostearic acid, licanic acid, arachidonic acid, arachidic acid, eicosenoic acid, eicosapentaenoic acid, behenic acid, erucic acid, docosahexaenoic acid, lignoceric acid, non-glyceryl esters thereof, and mixtures thereof.

In a particularly preferred embodiment of the present invention, the fatty acid material is selected from lauric acid, lauroleic acid, myristic acid, myristoleic acid, pentadecanoic acid, palmitic acid, palmitoleic acid, margaric acid, stearic acid, dihydroxystearic acid, oleic acid, ricinoleic acid, elaidic acid, linoleic acid, alpha-linolenic acid, dihomogamma-linolenic acid, eleostearic acid, licanic acid, arachidonic acid, arachidic acid, eicosenoic acid, behenic acid, erucic acid, lignoceric acid, non-glyceryl esters thereof, and mixtures thereof. Additionally, particularly preferred fatty acid materials include oleic acid, linoleic acid, esters thereof, and mixtures thereof.

The most preferred long chain fatty acids are linoleic acid and palmitic acid.

In a particular embodiment, linoleic acid and/or palmitic acid are used in combination with sodium salt of valeric acid.

As examples of long chain fatty acids, mention may be made of palmitic acid sold by Cayman Chemical (Ref. 10006627) and linoleic acid sold by Sigma (Ref. L1376).

The long chain fatty acids according to the present invention are used in an amount representing from 0,001 to 5% by weight relative to the total weight of the composition, preferably in an amount representing from 0,001% to 1% by weight relative to the total weight of the composition.

In a preferred embodiment, the ratio by weight of SCFA to LCFA (SCFA/LCFA) is ranging from 16 to 100.

### Dry skin

Dry skin essentially manifests itself through a feeling of discomforts such as tautness and/or of tension. Said dry skin is also rough to the touch and/or appears to be covered with squames. When the skin is slightly dry, the squames are abundant but barely visible to the naked eye. When this disorder worsens, they become fewer and fewer in number but increasingly visible to the naked eye.

The cause of dryness of the skin may be of constitutional or acquired type.

In one particular embodiment, the SCFA according to the invention is used to treat constitutional non-pathological dry skins or acquired non-pathological dry skins.

In the case of acquired dry skin, the involvement of outside parameters such as exposure to chemical agents, to difficult climatic conditions or to sunlight, alternatively certain therapeutic treatments (retinoids, for example) is determinant. Under these outside influences, the skin may then become momentarily and locally dry.

Non-pathological constitutional dry skin is dry skin for which the severity can depend on the outside factors already indicated. Senile skin (characterized by a general decrease in metabolism in the skin with age), fragile skin (very sensitive to outside factors and often accompanied by erythema and rosacea) and common xerosis (of probable genetic origin and manifesting itself predominantly on the face, the limbs and the back of the hands) enter into this skin category.

The SCFA according to the invention is thus found to be particularly effective for preventing and/or treating dry skin, and more particularly acquired dry skin and/or constitutional dry skin.

Non-pathological acquired constitutional dry skins can be characterized by a deficiency in lipids constituting the barrier and/or the aqueous-lipid film, in particular an endogenous insufficiency of sebum production by the sebaceous glands.

As mentioned above a sebum content of less than 100 µg/cm², measured at the T zone of the face, by the method described in FR 2 368 708, can be considered to be characteristic of hyposeborrheic dry skin.

The SCFA according to the invention is thus found to be particularly effective for preventing and/or treating hyposeborrheic dry skin

### Aged skin

The SCFA according to the invention is found to be particularly effective for preventing and/or treating aged skin, and more particularly hyposeborrheic aged skin. As mentioned above, premature aging is known as being attributed to the absence or insufficiency of sebum on the skin surface.

The term "aged skin" is intended to mean a general esthetic condition of the skin resulting from chronological aging and/or photo-induced aging.

More particularly, the present invention is directed toward preventing and/or reducing and/or treating signs of skin aging.

The expression "signs of skin aging" is intended to mean any of the modifications of the external appearance of the skin due to aging which is of chronological and/or photo-induced origin.

By way of example of this modification considered in the invention, mention may be made of a surface which is not very homogeneous and is less smooth, an epidermis which is thinned, wrinkles and fine lines, withered skin, a lack of elasticity and/or of tonicity of the skin, which leads to the appearance of flaccid and wrinkled skin.

In particular, the signs of skin aging that are targeted by the invention are chosen from thinning of the skin, a loss of firmness, a loss of elasticity, a loss of density or a loss of tonicity of the skin, an alteration of the surface appearance of the skin, the appearance of a marked microrelief of the skin, the appearance of roughness, the formation and/or presence of fine lines and/or of wrinkles, a modification of the radiance of the skin complexion, a wizened appearance of the skin, sagging of the skin, or withering of the skin.

Preferably, the signs of skin aging that are targeted by the invention are chosen from thinning of the skin, the appearance of a marked microrelief of the skin, the formation and/or the presence of fine lines and/or wrinkles, sagging of the skin and withering of the skin.

More preferably, the signs of skin aging that are targeted by the invention are chosen from the appearance of a marked microrelief of the skin, the formation and/or the presence of fine lines and/or wrinkles, sagging of the skin and withering of the skin.

### Cosmetic method

The present invention also refers to a cosmetic method for preventing and/or treating dry skin and/or aged skin, in particular hyposeborrheic dry skin and/or aged skin, comprising the application of a cosmetic composition on the skin, wherein the said composition comprises a conditioned culture medium obtained from at least one microorganism of the species *Propionibacterium acnes,* the said medium comprising at least one short chain fatty acid comprising from 3 to 8 carbon atoms, salts thereof, esters thereof and mixtures thereof.

Advantageously, the cosmetic methods for preventing and/or treating dry skin and/or aged skin according to the invention comprise the application of a cosmetic composition on a skin in need thereof.

The conditioned culture medium according to the invention is defined in the previous sections *"short chain fatty acids (SCFA)".*

The skin on which the cosmetic composition is applied is defined in the previous section *"Dry skin"* and *"Aged skin".*

The cosmetic composition comprises an effective amount of a short chain fatty acid (SCFA) comprising from 3 to 8 carbon atoms, salts thereof, esters thereof and mixtures thereof.

Advantageously, the short chain fatty acids according to the present invention are present in an amount representing from 0,01% to 5% by weight relative to the total weight of the composition, preferably in an amount representing from 0,025% to 0,15% by weight relative to the total weight of the composition.

As used herein, the terms "cosmetic composition" means a composition suitable for an application on the skin, in particular a composition which comprises a physiologically acceptable medium.

The terms "physiologically acceptable medium" means a medium that is suitable for the topical administration of a composition, i.e. that is compatible (not toxic) with the skin of the face, the body and the scalp.

### Dosage forms

The cosmetic composition may be in any of the dosage forms conventionally used for this type of application and particularly in the form of aqueous gels, aqueous or hydroalcoholic solutions. They may also, by the addition of a fatty or oil phase, be in the form of dispersions such as lotion, emulsions of liquid or semi-liquid consistency such as milk, obtained by dispersing a fatty phase in an aqueous phase (O/W) or conversely (W/O), or suspensions or emulsions of soft, semi-solid or solid consistency such as cream or gel, or multiple emulsions (W/O/W or O/W/O), microemulsions, ionic and/or non-ionic type vesicle dispersions, or wax/aqueous phase dispersions. These compositions are prepared using usual methods.

### Aqueous phase

The compositions according to the invention intended for cosmetic use may comprise at least one aqueous phase. They are formulated particularly in aqueous lotions or in a water-in-oil emulsion, an oil-in-water emulsion, or in a multiple emulsion (triple oil-in-water-in-oil or water-in-oil-in-water emulsion) (such emulsions are known and described for example by C. FOX in "Cosmetics and Toiletries" - November 1986 - Vol 101 - pages 101-112).

The aqueous phase of said compositions contains water and generally other solvents soluble in water or miscible with water. Solvents that are soluble or miscible in water comprise short-chain mono alcohols, for example in C₁-C₄ such as ethanol, isopropanol; diols or polyols.

The compositions according to the invention preferably have a pH ranging from 3 to 9 depending on the substrate chosen.

When the composition(s) is (are) in the form of emulsion, it (they) generally contain(s) one several emulsifying surfactants, depending on the nature of the emulsion.

The total quantity of emulsifiers in the composition(s) according to the invention shall preferably be at contents in active material ranging between 1 and 8% by weight and more particularly between 2 and 6% by weight relative to the total weight of the composition.

### Fatty phase

The compositions according to the invention may contain at least one organic liquid phase non-miscible in water, known as a fatty phase. This generally includes one or several hydrophobic compounds rendering said phase non-miscible in water. Said phase is liquid (in the absence of a structuring agent) at room temperature (20-25°C). Preferentially, the organic liquid phase non-miscible in water according to the invention generally comprises at least one volatile oil and/or one non-volatile oil and optionally at least one structuring agent.

"Oil" herein means a fatty body that is liquid at room temperature (25°C) and atmospheric pressure (760mm Hg namely 1.05x10⁵ Pa).

The oil may be chosen from all physiologically acceptable and particularly cosmetically acceptable oils, in particular mineral, animal, plant, synthetic oils; in particular, volatile or non-volatile hydrocarbon and/or silicone and/or fluorinated oils and mixtures thereof.

By way of examples of oils suitable for use in the invention, mention may be made of:
i) hydrocarbon plant oils such as liquid triglycerides of fatty acids with 4 to 24 carbon atoms such as triglycerides of caprylic/capric acids such as those sold by the Stearineries Dubois Company or those sold under the names Miglyol 810, 812 and 818 by the Dynamit Nobel Company, jojoba oil;
ii) linear or branched hydrocarbons of mineral or synthetic origin, such as paraffin oils and derivatives thereof, petroleum jelly, polydecenes, polybutenes, hydrogenated polyisobutene such as Parleam, squalane;
iii) synthetic ethers having from 10 to 40 carbon atoms;
iv) synthetic esters such as isononyl isononanoate, isopropyl myristate, isopropyl palmitate, C₁₂ to C₁₅ alcohol benzoate
v) silicone oils such as non-volatile polydimethylsiloxanes (PDMS), either linear (dimethicones) or cyclic (cyclomethicones).

The compositions according to the invention may further comprise one or several cosmetic additives chosen among softeners, opacifiers, stabilizers, preservatives, perfumes, a fatty phase structuring agent chosen particularly from waxes, pasty compounds, gelling agents; organic or inorganic fillers; thickening or suspension agents, or any other ingredient conventionally used in cosmetics for this type of application.

Obviously, the skilled person will take care to choose such optional compound(s) in such a way that the advantageous properties intrinsically associated with the composition according to the invention are not altered, or are not substantially altered, by the envisaged additive(s).

These optional additives may be present in the composition at 0.001 to 80% by weight, in particular from 0.1 to 40% by weight, with respect to the total weight of the composition.

These additives, depending on their nature, may be introduced in the fatty phase or in the aqueous phase of the composition, or in lipid vesicles. In any case, these additives and their proportions shall be chosen by the skilled person such that the advantageous properties of the mixture according to the invention are not altered or not substantially altered by the envisaged additives.

The composition according to the invention may further comprise other active constituents such as desquamating agents, depigmenting or propigmenting agents, anti-glycation agents, anti-inflammation or soothing agents, agents that stimulate the synthesis of dermal or epidermal macromolecules and/or prevent their degradation, agents that stimulate the proliferation of fibroblasts and/or keratinocytes or that stimulate differentiation of keratinocytes, dermo-relaxing agents, tensor agents, agents acting on microcirculation, agents acting on the energy metabolism of cells, UV filters, odor absorbing agents, or mixtures thereof.

When the composition is an emulsion, the proportion of the fatty phase may be between 5 and 80% by weight, preferably between 8 and 50% by weight relative to the total weight of the composition. The emulsifier and the co-emulsifier may be present in a proportion ranging from 0.3% to 30% by weight, preferably from 0.5% to 20% by weight, relative to the total weight of the composition.

The composition according to the invention can be more or less fluid and have the appearance of a white or colored cream, of an ointment, of a milk, of a lotion, of a serum, of a paste, of a gel or of a foam. It can possibly by applied in the form of an aerosol. It can also be in solid form, particularly in the form of a stick. It can also be on a support, for example on wipes such as makeup removal wipes.

The composition according to the invention may form a skin care composition, particularly a cleaning, protection, treatment or care cream for the face, hands or body, such as day creams, night creams, makeup removal creams, foundation creams, sunscreen creams, makeup removal milk, a body protection or care milk, a sunscreen milk, a lotion, gel or foam for skin care such as a makeup lotion.

In the whole description, the expression «comprising a» or «containing a» means «comprising at least one» or «containing at least one», unless otherwise specified.

In the description and in the following examples, unless stated otherwise, the percentages are percentages by weight and the ranges of values written as "between ... and ..." include the upper and lower limits specified. The ingredients are mixed, before their shaping, in order and under conditions easily determined by the skilled person.

The present invention will be further illustrated by the figures and examples below.

### Figures

Figure 1: Overview of SCFAs (C3, C4, and C5) which induce de-novo production of lipid droplets & mediate holocrine secretion (top scheme). This phenomenon is maintained during co-treatment with LCFAs aimed at boosting the sebogenic response (bottom scheme).
Figure 2: Effect of SCFA propionate on lipid droplets per cell.
Figure 3 : Effect of SCFA butyrate on lipid droplets per cell.
Figure 4: Effect of SCFA valerate on lipid droplets per cell.
Figure 5: Effect of SCFA propionate on size of LDs.
Figure 6: Effect of SCFA butyrate on size of LDs.
Figure 7: Effect of SCFA valerate on size of LDs.
Figure 8: Effect of SCFA propionate on intensity of LDs.
Figure 9: Effect of SCFA butyrate on intensity of LDs.
Figure 10: Effect of SCFA valerate on intensity of LDs.
Figure 11: Effect of SCFA propionate on the number of nuclei.
Figure 12: Effect of SCFA butyrate on the number of nuclei.
Figure 13: Effect of SCFA valerate on the number of nuclei.
Figure 14: Effect of SCFA propionate on size of the nucleus.
Figure 15: Effect of SCFA butyrate on size of the nucleus.
Figure 16: Effect of SCFA valerate on size of the nucleus.
Figure 17: Growth curves of *Propionibacterium acnes:* (Optical Density (OD) and Colony Forming Units (CFU).
Figure 18: Quantification of propionate in bacterial culture supernatants.
Figure 19: Effect of bacterial supernatants on lipid droplets per cell.
Figure 20: Effect of bacterial supernatants on number and size of the nucleus.
Figure 21: Fold change of triglycerides (TGs) produced and secreted in cell culture media by non-treated sebocytes (Control: Ctrl.) and sebocytes treated with propionate (P) at 5 (P5) and 15mmol.I-1 (P15), butyrate (B) at 5 and 15mmol.I-1 and valerate (V) at 5 and 15mmol.I-1, after 24h of incubation.
Figure 22: The additive effect of combining one SCFA, valerate (V) with palmitic acid (PA) on size of lipid droplets (LDs).
Figure 23: The additive effect of combining one SCFA, valerate (V) with linoleic acid (LA) on size of lipid droplets (LDs).
Figure 24: The additive effect of combining one SCFA, valerate (V) with palmitic acid (PA) on intensity of lipid droplets (LDs).
Figure 25: The additive effect of combining one SCFA, valerate (V) with linoleic acid (LA) on intensity of lipid droplets (LDs).

### Examples

### Example 1 - Demonstration of the lipids production and sebum secretion effects of SCFA according to the invention and comparative test with sodium acetate (outside of the invention)

### A) Material and Methods

**Bacteria culture.** Stored at -80°C, Strains of *Propionibacterium acnes* ATCC 6919 were pre-cultured on agar plate (Trypcase Soy Agar, bioMérieux, cat. 41466) incubated in anaerobic bag (GENbag, bioMérieux, cat. 45534, controlled with Anaerotest strip, Merck, cat. 1.15112) for 5 days at 33°C. Single isolated colonies were sampled and pre-cultured in liquid medium in triplicate in 9 ml of Trypcase Soy Broth (bioMérieux, cat. 42100) and incubated under anaerobic conditions (anaerobic bag controlled with Anaerotest strip) for 5 days at 33°C. Each replicate of liquid pre-culture was used to inoculate three flasks of Trypcase Soy Broth supplemented with 2% glycerol (Promega, cat. H5433) at OD 0.05 ± 0.002 (λabs= 600 nm, Shimadzu UV-1800) and then aliquoted in snap cap tubes (Greiner, cat. 187262) and incubated at 33°C under anaerobic condition. Growth curves were determined by measuring optical density and CFU (Colony Forming Units: serial dilutions plated on trypcase soy agar plate incubated 48 hours at 33°C under anaerobic condition).

**Quantification of propionate in supernatants.** Propionate of supernatants from bacterial culture was extracted using an aliquot of 500 µl acidified with 1% formic acid (Merck, cat. 1.00264) following a ratio of 1:10 (formic acid:supernatant), vortexed for 1 min and stored at 4°C. Supernatants were analyzed by GC-FID using an Agilent DB-FFAP column.

**Cell culture.** Vials containing 3T3 mitomycine treated cells stored in 20% DMSO (Dimethylsulfoxide, Sigma Ref. D2650) in liquid nitrogen have been thawed in a water bath at 37°C and directly washed with prewarmed G7F culture media (Table 1). 3T3m cells were plated at 40.10³ cells.cm² in a 175 cm² flask (Thermoscientific Ref. 159910) and incubated 2 hours at 37°C, 5% CO₂. Sebocytes were obtained from primary cell line and stored in 10% DMSO in liquid nitrogen. Vials containing sebocytes were thawed in a water bath at 37°C and directly washed with prewarmed G7F culture media. Sebocytes were plated at 2600 cells.cm² in a 175 cm² flask containing attached 3T3m cells and incubated (37°C, 5% CO₂) to reach 70-80% confluence. After 5 days, cells were harvested using trypsin (Gibco Ref. 25300) and transferred in 96 well plates (10x10³ cells per well, Greiner Bio-one Ref. 655956) and incubated (37°C, 5% CO₂).

After sampling of aliquots for OD and CFU measurements, tubes were closed and immediately centrifuged (6400 g, 5min) at room temperature (centrifuge Eppendorf 5810R). Supernatants were then aspirated in a 10 ml syringle (Thermo Ref. SS+10I), filtered through a 0.45 µm, 25 mm (Pall Ref. 4614) filter and stored directly at -20°C.

**Table 1. Composition of G7F media.**

| Compounds | Commercial reference | Concentration |
|---|---|---|
| DMEM | Gibco Ref. 10569 | ~67% |
| Ham's F-12 Nutrient Mix | Gibco Ref. 31765 | 22% |
| FetalClone II Serum | HyClone Ref. SH30066.03 | 10% |
| Antibiotic-Antimycotic (100x) | Gibco Ref. 15240062 | 1% |
| Supplemented with: | | |
| Insulin solution human | Sigma Ref. I9278 | 0.00025% |
| 3,3',5-Triiodo-L-thyronine sodium salt | Sigma Ref. T2752 | 0.05% |
| Hydrocortisone 21-hemisuccinate sodium salt | Sigma Ref. H4881 | 2x10⁻⁸% |
| apo-Transferrin human | Sigma Ref. T2252 | 0.0001% |
| (-)-Isoproterenol hydrochloride | Sigma Ref. I6504 | 0.05% |
| Adenine hydrochloride hydrate | Sigma Ref. A9795 | 0.0001% |
| Epidermal Growth factor | Sigma Ref. GF144 | 0.004% |

**Cell treatment.** After 24 hours, cell media was replaced by modified cell media (G7F w/o hydrocortisone and w/o antibiotic-antimycotic) supplemented with: modified cell media (control), sodium acetate (CH₃COONa, Sigma Ref. 32319), sodium propionate (CH₃CH₂COONa, Sigma Ref. P1880), sodium butyrate (CH₃(CH₂)₂COONa Sigma Ref. 303410), valeric acid (CH₃(CH₂)₃COOH, Sigma Ref. 75054). For valeric acid, the pH in modified cell media was neutralized with NaOH (Sodium hydroxide, Sigma 283060). Palmitic acid (C16:0, Cayman Ref. 10006627) and linoleic acid (C18:2 cis 9,12, Sigma Ref. L1376) were both dissolved in pure ethanol (Merck Ref. 1.00983) for a final concentration per well of 60 µmol.l⁻¹. For these treatments, modified cell media supplemented with pure ethanol was used as controls. Supernatants (25%) sampled in exponential and stationary phases were neutralized with NaOH and were controlled with modified cell media supplemented at 25% with pure bacterial culture media. All conditions were repeated 8 times (n=8) for SCFA experiments and 24 times (n=24) for supernatants assays. Microplates were incubated 48 hours (37°C, 5% CO2) and directly sampled and/or stained and analyzed.

**Triglycerides quantification.** Assays have been realized using Triglycerides Assays Kit - Quantification (Abcam, ref. Ab65336) following manufacturer's instructions. Each supernatant of the 8 replicates have been sampled (50 µl) and analyzed following the colorimetric detection method of the kit (OD 570 nm) using a plate reader Infinite 200PRO (Tecan).

**Cell staining.** Wells were washed with DPBS solution (Dulbecco's Phosphate-Buffered Saline, Gibco Ref. 14040) and were fixed with 10% Formalin (Leica Ref. 3800598). Lipid droplets were stained with BODIPY (Invitrogen Ref. D3922) diluted in DPBS solution supplemented with delipidated BSA (Bovin Serum Albumin, Sigma Ref. A7906). Nuclei were stained with Hoechst (Invitrogen, Ref. H3570) diluted in DPBS solution.

**High content imaging, images analysis and statistics.** An inverted automated epifluorescent microscope (ImageXpress Micro 4, Molecular Devices) was used to acquire 16 images of each well with a 10x objective (well coverage = 80%) controlled by MetaXpress software (version 6.2.3.733, Molecular Devices). Images were analyzed using the MetaXpress image based module Transfluor (Molecular Devices). Results were exported into GraphPad Prism (version 7.01) to determine statistical significance using One-way ANOVA and expressed on bar graph as mean ± SD or on box plot ± min and max. *: P-value < 0.05, **: P-value < 0.01, ***: P-value < 0.001, ****: P-value < 0.0001.

### B) Results

### a. Effect of SCFA on lipid droplets (lipids production) and holocrine secretion (sebum secretion) by high content screening (HCS)

Sebocytes were seeded in 96 well plates, followed by treatment with SCFA and/or LCFAs with at least 8 replicate wells (1 column) for each condition. Sebogenesis (lipid formation) and holocrine secretion (release of lipids) have been analyzed by imaging the cell layer using a high content screening (HCS) system in all 8 wells (ensuring 80% coverage of the cell layer in each well) by characterization of 5 different parameters: number of nucleus, size of nucleus, average number of Lipid Droplets (LDs) per cell, average size of LDs and average intensity of LDs.

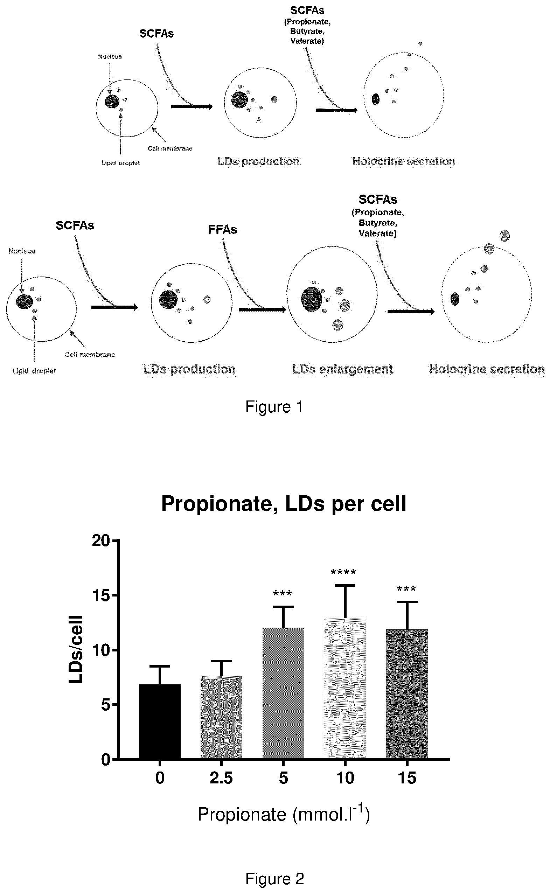

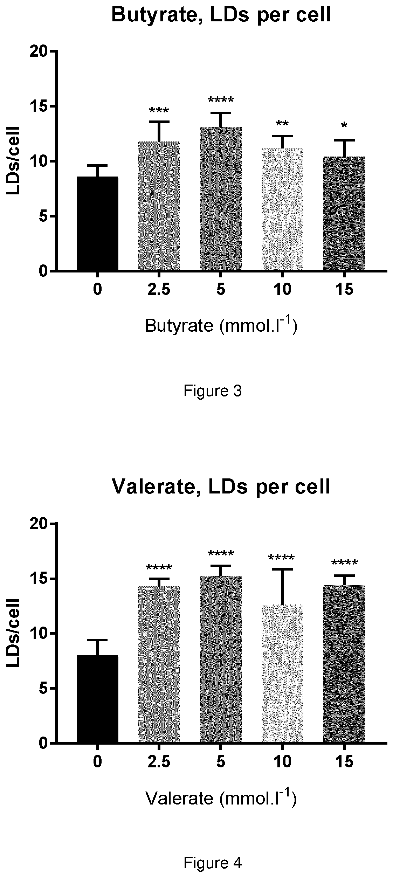

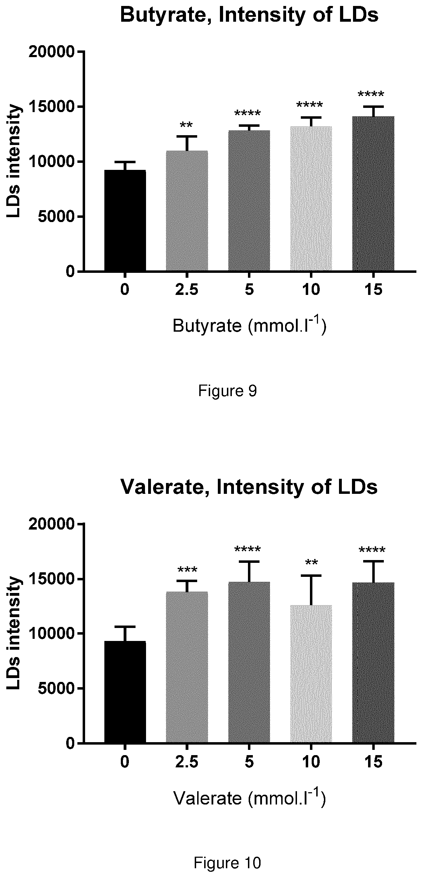

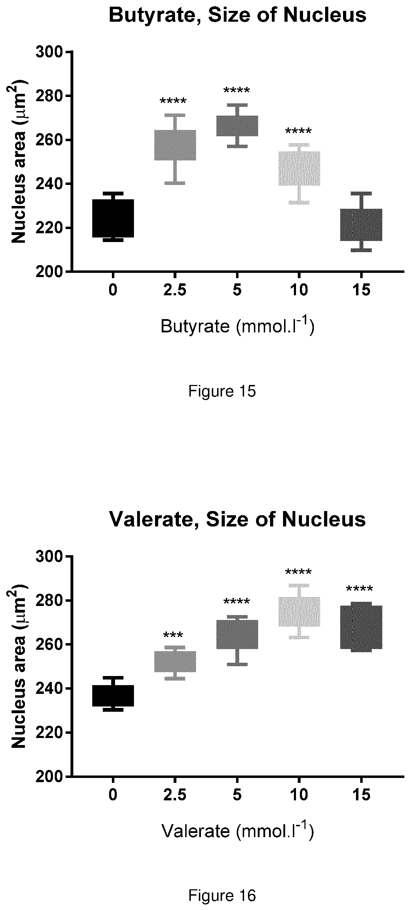

3 SCFA as sodium salts: propionate (CH₃CH₂COOH), butyrate (CH₃(CH₂)₂COOH) and valerate (CH₃(CH₂)₃COOH) were tested at 4 different concentrations (2.5, 5, 10 and 15 mmol.l⁻¹). Results are presented in Figures 2 to 16.

### a.1 Effect of SCFA on lipids droplets (lipids production)

### a.1.i Effect of SCFA on number of lipid droplets (LDs)

Compared to the control, number of LDs per cell is significantly higher and equivalent for all the conditions tested. Interestingly, the maximum *de novo* synthesis of LDs was the same for all of the conditions tested (see Figures 2 to 4).

### a. 1.ii Effect of SCFA on size of lipid droplets (LDs)

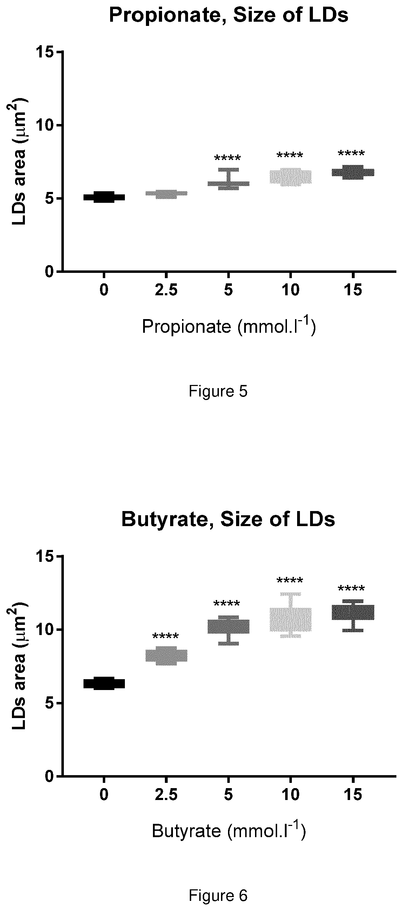

Compared to controls, the size of LDs is higher for all of the conditions tested. LDs in cells treated with propionate are the smallest but LDs increased in size as the concentration of propionate increased. This trend is enhanced with butyrate and valerate (e.g. size of LDs for valerate treated cells with 15 mmol.l⁻¹ > 10 > 5 > 2.5 mmol.l⁻¹, P-value < 0.001) (see Figures 5 to 7).

### a. 1.iii Effect of SCFA on intensity of lipid droplets (LDs)

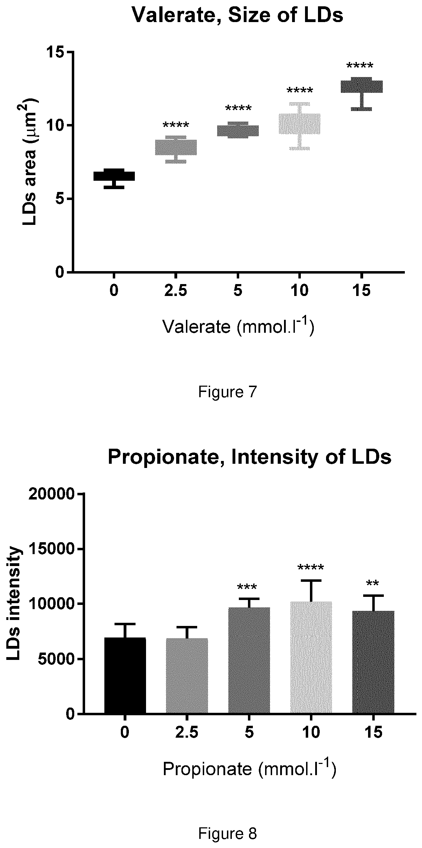

Based on the principle of BODIPY staining (hydrophobic-hydrophobic interactions), fluorescence intensity of lipid droplets can be related to the quantity of lipid embedded within the droplets. Fluorescence of LDs in cells treated with SCFA is more intense compared to the control (see Figures 8 to 10).

### a.2 Effect of SCFA on holocrine secretion (sebum secretion)

### a.2.i Effect of SCFA on nuclear count

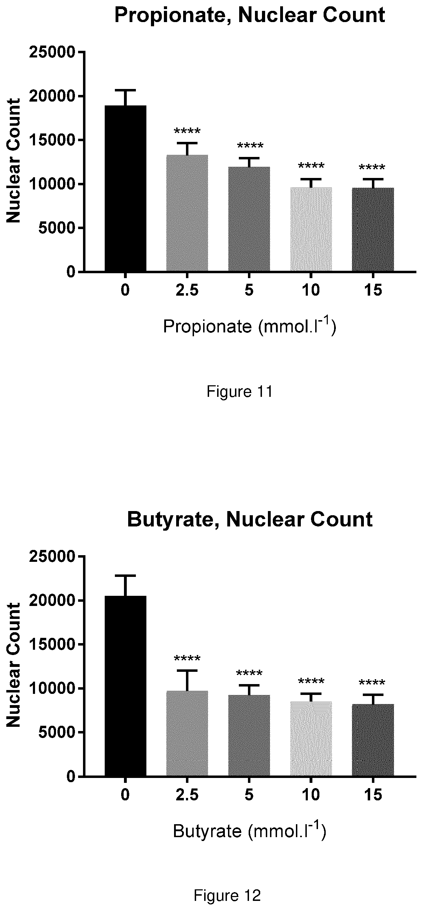

Holocrine secretion is the final step of sebogenic process wherein the mature cells burst to release the lipid contents (Schneider and Paus, 2010). Holocrine secretion is thus associated with a decrease in the nuclear count per well. This trend is clearly observable for cells treated with propionate, butyrate and valerate. (see Figures 11 to 13).

In order to demonstrate that the decrease of cell number is due to holocrine secretion and not toxicity, we correlated these observations with the size of nucleus and also estimated the triglycerides in the supernatant (see below).

### a.2.ii Effect of SCFA on size of nucleus

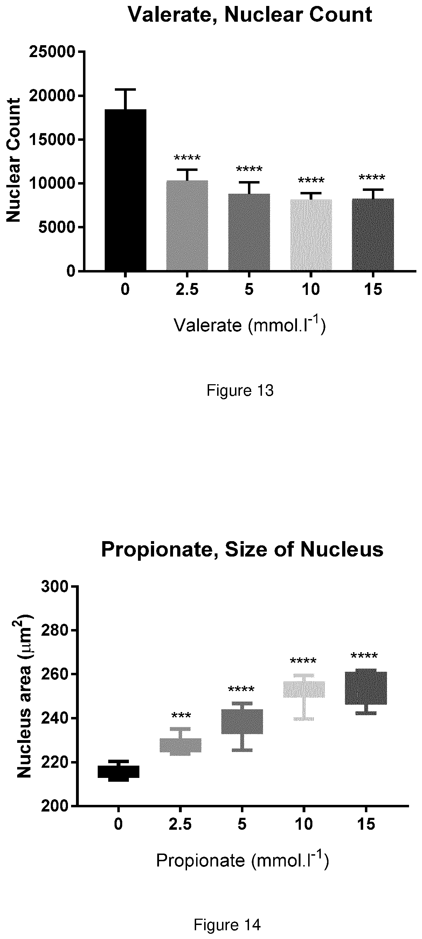

Holocrine secretion involves maturation of sebocyte cells to a terminal bursting stage. This process involves enlargement of the nuclei during the expansion phase and final shrinkage just before the sebocyte cells burst to release the lipid content (Tosti, 1974). This trend is clearly observed in cells treated with the 3 SCFA. (see Figures 14 to 16).

### a.3 Effect of the conditioned culture medium (supernatants) of P. acnes ATCC 6919 containing SCFA on lipids droplets (lipids production) and holocrine secretion (sebum secretion)

### a.3.i Effect of P. acnes supernatants containing SCFA on number of lipid droplets (LDs) (lipids production)

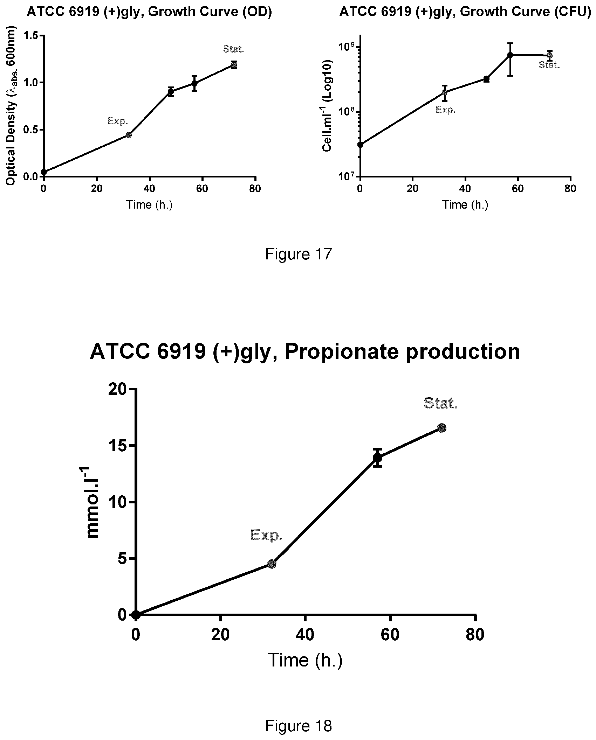

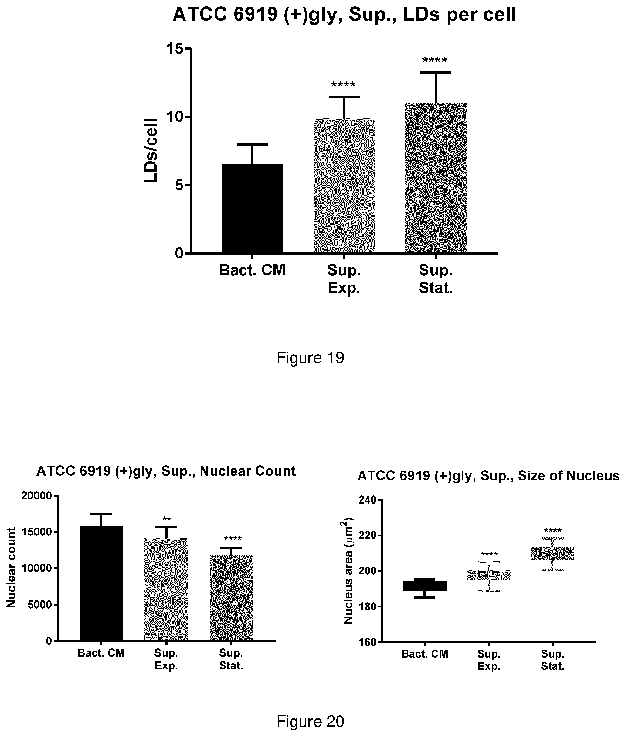

Treated cells with bacterial supernatants (sampled during exponential (Sup. Exp.) and stationary (Sup. Stat.) phases) and containing propionate as the SCFA (see Figure 17 and 18) have been compared to cells treated with the media used to grow bacteria (Bacterial Culture Media, Bact. CM). Compared to the control, number of LDs per cell is significantly higher for all the conditions tested. Moreover, number of LDs per cell increases with concentration of propionate produced by bacteria (Sup. Stat. > Sup. Exp.). Interestingly number of LDs per cell of sebocytes treated with the supernatants sampled in stationary phase (4.2 mmol.l⁻¹ of propionate, about 11 LDs/cell) is very close to the number of LDs measured for approximately the same concentration of pure propionate (5 mmol.l⁻¹) used to treat the sebocytes in sections a1 and a2 (about 12 LDs/cell, Figure 19). Treating cells with bacterial supernatants containing SCFAs induces de novo production of LDs (see Figure 19).

### a.3.ii Effect of P. acnes supernatants containing SCFA on nuclear size and nuclear count (sebum secretion)

The number of nuclei in the wells treated with bacterial supernatants decreases. (see Figure 20).

This diminution is even more important for cells treated with Sup. Stat. and is comparable to the nuclear count measured for approximately the same concentration of pure propionate (5 mmol.l⁻¹) used to treat the sebocytes in Figure 11.

This is correlated with the clear trend of nuclear size enlargement for sebocytes treated with bacterial supernatants (see Figure 20).

We conclude that supernatants containing at least one SCFA have a positive effect on holocrine secretion in sebocytes (see Figure 20).

**Conclusion:** treatment of sebocytes with SCFA or supernatants containing SCFA has multiple effects:
1) SCFA lead to *de novo* production of lipid droplets (Figures 2 to 16).
2) Butyrate as well as valerate treatment has a positive effect on the size of lipid droplets (Figures 6 and 7).
3) Propionate, butyrate as well as valerate treatment boost sebum production process by promoting holocrine secretion (Figures 11 to 16).
4) Treatment of sebocytes with bacterial supernatants containing SCFA has similar effects on *de novo* production of LDs/sebum production (Figure 19), and holocrine secretion (Figure 20).

### b. Effect of SCFA on holocrine secretion (sebum secretion) by quantifying secreted triglycerides (TGs) and comparison with acetate (SCFA that does not mediate holocrine secretion)

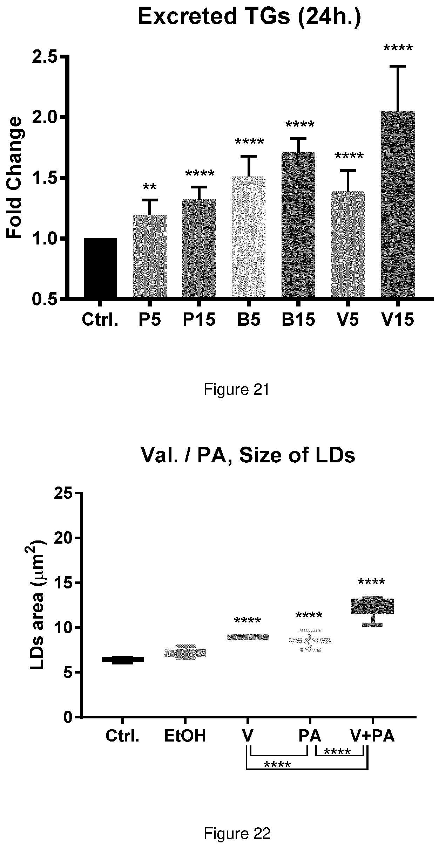

Holocrine secretion is the final step of sebogenic process wherein the mature cells burst to release the lipid contents. Promotion of holocrine secretion by SCFA was based on observations and interpretations of a lower number of cells in association with an enlargement of nuclear area. In order to demonstrate the induction of holocrine secretion on treatment with SCFA, the secreted triglycerides (TGs, the major lipid class of sebum) in sebocyte supernatants treated with 5 and 15 mmol⁻¹ of SCFA was quantified. Results are presented in Figure 21.

In the supernatants of the sebocytes treated with SCFAs, the fold change (FC) is significantly higher for all the conditions tested (min. FC ~ 1.2 (P5): +20% of TGs excreted by cells treated with P5, max. FC ~ 2 (V15): +100% of TGs excreted by cells treated with V15). Moreover, the quantity of TGs produced and excreted is systematically higher when concentration increases (e.g. FC of TGs in B15 > FC of TGs in B5, P-value = 0.02). This clearly establishes that SCFAs mediate holocrine secretion in sebocyte cells.

### b.1 Comparative outside of the invention (SCFA = acetate)

TGs secreted in supernatants of propionate and acetate treated sebocytes was compared: while both acetate and propionate lead to similar sebogenic response (based on lipid induction parameters), no apparent change in nuclear parameters was observed for acetate treated cells. These results are presented Table 2 below.

Compared to the control, the effect of acetate on *de novo* lipid induction is significant and, similar to propionate (same number of LDs per cell, similar size, similar intensity).

However, no effect of acetate is observed on the nuclear count (same number of cells compared to the control), and the size of nucleus (similar size as compared to the control), which is in sharp contrast with propionate treated cells. This would indicate that while same quantity of lipids is produced by cells treated with both acetate and propionate, only sebocytes treated with propionate would mediate holocrine secretion **and release the produced sebum** in the supernatant. Interestingly, TG estimation from supernatants of acetate and propionate treated cells clearly supported these observations, further establishing a unique role of propionate, holocrine secretion in sebocyte cells.

**Table 2. Comparison between acetate and propionate on lipid induction and holocrine secretion effect.**

| | | Ctrl. | **Acetate (5 mmol.L^{- 1}) (A5) outside of the invention** | **Proprionate (5 mmol.L⁻¹) (P5) according to the invention** |
|---|---|---|---|---|
| Lipid induction | LDs per cell | 6.85±1.68 | 11.97±2.08 (****) **↑** | 12.04±1.92 (***) **↑** |
| | Size of LDs | 5.06±0.20 | 5.86±0.31 (****) **↑** | 6.08±0.38 (****) **↑** |
| | Intensity of LDs | 6962±1193 | 10251±1295 (****) **↑** | 9656±798 (***) **↑** |
| Holocrine | Nuclear count | 18905±1745 | **18574±2148 =** | **11921±1018** (****) **↓** |

| secretion | Size of nucleus | 216±3 | **215±5 =** | **238±7 (****) ↑** |
|---|---|---|---|---|
| | FC (compared to control) | 1 | FC = 0.90, = | FC = 1.2, **↑** |
| | % of excreted TGs (compared to control) | - | -10% *=* | +20% (**) **↑** |

| | | | | |
|---|---|---|---|---|
| *Results are presented as mean±SD and fold change (FC) plus % for excreted TGs.* **↑*****:** significantly higher than the control,* **↓:** *significantly lower than the control,* =: *no significant difference with the control.* | | | | |

**Conclusion:** The significant increase of TGs secreted by sebocytes cultured during 24 h. in media supplemented with SCFA, correlated with the trend seen for nuclear parameters, and demonstrates that SCFA boost the sebum production process by mediating both lipid production and promoting holocrine secretion.

### c. Effect of LCFA in combination with SCFA on lipids droplets (lipids production)

One SCFA, valerate, V (CH3(CH2)3COOH), was tested in association with two LCFA: palmitic acid, PA (C16:0) and linoleic acid, LA (C18:2 cis 9,12) in two experiments to demonstrate an additive effect on sebum production. Results are presented in Figure 22 to 25.

### c.1 Effect of LCFA in combination with SCFA on size of lipids droplets (LDs)

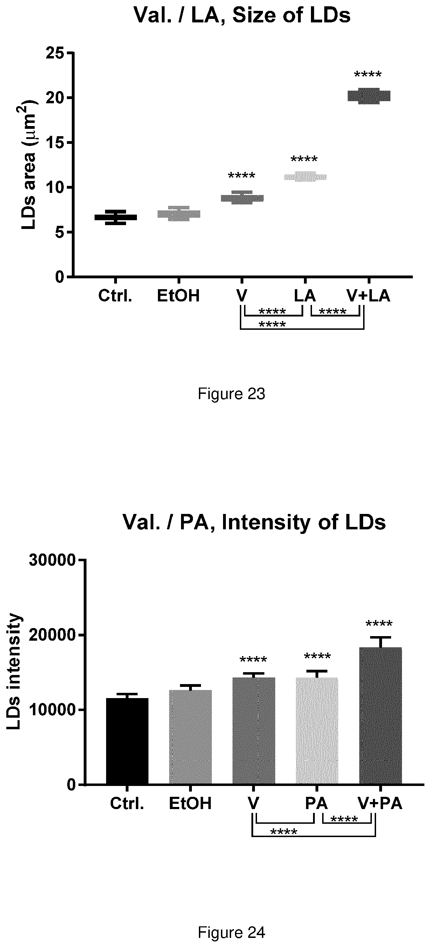

When palmitic acid was combined with valerate, the LDs were bigger than when palmitic acid was used alone. This boost effect is even more noticeable with valerate and linoleic acid (Figures 22 and 23).

### c.2 Effect of LCFA in combination with SCFA on intensity of lipids droplets (LDs)

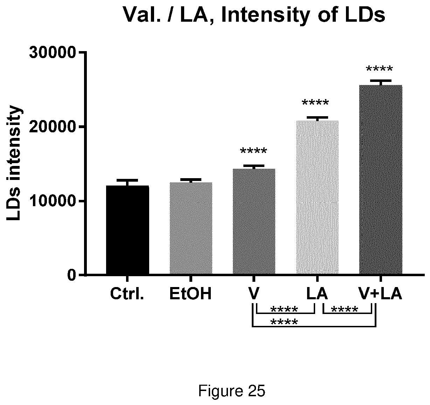

The fluorescence of LDs in cells treated with valerate and/or the two LCFA follows the same conclusion, that there is a better effect on intensity of lipid droplets when LCFA is added in combination with valerate (see Figures 24 and 25).
**Conclusion:** The combination of SCFA and LCFA has an additive effect on the size of lipid droplets (Figures 24 and 25).

### Example 2 - skin care composition

The composition as described below is prepared.

| **Compounds** | **Concentration** (w/w) |
|---|---|
| Sodium propionate | 0,01 |
| Palmitic acid | 1 |
| Sodium methyl stearoyl taurate | 0,23 |
| Xanthan gum | 0,05 |
| carbomer | 0,2 |
| water | qs 100 |

The composition is applied on the skin and provides a decrease of signs of skin dryness.

## Claims

1. Non-therapeutic cosmetic use of a conditioned culture medium obtained from at least one microorganism of the species *Propionibacterium acnes,* the said culture medium comprising at least one short chain fatty acid comprising from 3 to 8 carbon atoms, salts thereof, esters thereof and mixtures thereof for preventing and/or treating dry skin and/or aged skin, in particular hyposeborrheic dry skin and/or aged skin.

2. Cosmetic use according to claim 1, wherein the conditioned culture medium is comprised in a cosmetic composition.

3. Cosmetic use according to any one of preceding claims, wherein the at least one short chain fatty acid comprises a straight and saturated aliphatic chain containing 3 to 5 carbon atoms.

4. Cosmetic use according to claim 1 or 2, wherein the at least one short chain fatty acid is chosen from propionic acid, butyric acid, valeric acid, salts thereof, esters thereof and mixtures thereof.

5. Cosmetic use according to anyone of preceding claims, wherein the at least one short chain fatty acid is obtained from at least one microorganism of the strain *Propionibacterium acnes* ATCC 6919.

6. Cosmetic use according to claim 1, wherein the said conditioned culture medium is obtained by the process comprising the following steps:
i) culturing at least one microorganism of the species *Propionibacterium acnes* such as *Propionibacterium acnes* ATCC 6919;
ii) separating, in particular by centrifugation, the culture supernatant from the biomass;
iii) recovering the culture supernatant; and
iv) optionally stabilizing the culture supernantant for instance by filtration.

7. Cosmetic use of a conditioned culture medium as defined in anyone of claims 1 to 6, wherein the said conditioned culture medium is used in combination with at least one long chain fatty acid comprising at least 10 carbon atoms, non-glyceryl esters thereof, and mixtures thereof.

8. Cosmetic use according to anyone of preceding claims, wherein dry skin is **characterized by** presenting a feeling of discomforts such as tautness and/or tension, rough to the touch and/or squames.

9. Non-therapeutic cosmetic method for preventing and/or treating dry skin and/or aged skin, in particular hyposeborrheic dry skin and/or aged skin, comprising the application of a cosmetic composition on the skin, wherein the said composition comprises a conditioned culture medium as defined in anyone of claims 1 to 8.

## Patentansprüche

1. Nichttherapeutische kosmetische Verwendung eines aufbereiteten Kulturmediums, das aus zumindest einem Mikroorganismus der Gattung *Propionibacterium acnes* erhalten wurde, wobei das Kulturmedium zumindest eine kurzkettige Fettsäure mit 3 bis 8 Kohlenstoffatomen, Salze davon, Ester davon und Gemische davon umfasst, bei der Verhinderung und/oder Behandlung von trockener Haut und/oder gealterter Haut, insbesondere von hyposeborrhoischer trockener Haut und/oder gealterter Haut.

2. Kosmetische Verwendung nach Anspruch 1, wobei das aufbereitete Kulturmedium in einer kosmetischen Zusammensetzung umfasst ist.

3. Kosmetische Verwendung nach einem der vorstehenden Ansprüche, wobei die zumindest eine kurzkettige Fettsäure eine gerade und gesättigte aliphatische kette mit 3 bis 5 Kohlenstoffatomen umfasst.

4. Kosmetische Verwendung nach Anspruch 1 oder 2, wobei die zumindest eine kurzkettige Fettsäure aus Propionsäure, Buttersäure, Baldriansäure, Salzen davon, Estern davon und Gemischen davon ausgewählt ist.

5. Kosmetische Verwendung nach einem der vorstehenden Ansprüche, wobei die zumindest eine kurzkettige Fettsäure aus zumindest einem Mikroorganismus des Stammes *Propionibacterium acnes* ATCC 6919 erhalten wird.

6. Kosmetische Verwendung nach Anspruch 1, wobei das aufbereitete Kulturmedium mithilfe des Prozesses erhalten wird, der die folgenden Schritte umfasst:
i) Kultivieren zumindest eines Mikroorganismus der Gattung *Propionibacterium acnes* wie *Propionibacterium acnes* ATCC 6919;
ii) Abscheiden des Kulturüberstands von der Biomasse, insbesondere mithilfe von Zentrifugation;
iii) Gewinnen des Kulturüberstands; und
iv) optional Stabilisieren des Kulturüberstands, z. B. mithilfe von Filtration.

7. Kosmetische Verwendung eines aufbereiteten Kulturmediums nach einem der Ansprüche 1 bis 6, wobei das aufbereitete Kulturmedium in Kombination mit zumindest einer langkettigen Fettsäure mit zumindest 10 Kohlenstoffatomen, Nicht-Glyceryl-Estern davon und Gemischen davon verwendet wird.

8. Kosmetische Verwendung nach einem der vorstehenden Ansprüche, wobei trockene Haut **dadurch gekennzeichnet ist, dass** ein Gefühl von Unbehagen wie etwa Straffheit und/oder Spannung, raues Tastgefühl und/oder Schuppen vorliegen.

9. Nichttherapeutisches kosmetisches Verfahren zum Verhindern und/oder Behandeln von trockener Haut und/oder gealterter Haut, insbesondere von hyposeborrhoischer Haut und/oder gealterter Haut, umfassend das Auftragen einer kosmetischen Zusammensetzung auf die Haut, wobei die Zusammensetzung ein aufbereitetes Kulturmedium nach einem der Ansprüche 1 bis 8 umfasst.

## Revendications

1. Utilisation cosmétique non thérapeutique d'un milieu de culture conditionné obtenu à partir d'au moins un micro-organisme de l'espèce *Propionibacterium acnes,* ledit milieu de culture comprenant au moins un acide gras à chaîne courte comprenant de 3 à 8 atomes de carbone, des sels de celui-ci, des esters de celui-ci et des mélanges de ceux-ci pour la prévention et/ou le traitement de la peau sèche et/ou de la peau âgée, en particulier de la peau sèche et/ou de la peau âgée hyposéborrhéique.

2. Utilisation cosmétique selon la revendication 1, dans laquelle ledit milieu de culture conditionné est compris dans une composition cosmétique.

3. Utilisation cosmétique selon l'une quelconque des revendications précédentes, dans laquelle l'au moins un acide gras à chaîne courte comprend une chaîne aliphatique linéaire et saturée contenant de 3 à 5 atomes de carbone.

4. Utilisation cosmétique selon la revendication 1 ou 2, dans laquelle l'au moins un acide gras à chaîne courte est choisi parmi l'acide propionique, l'acide butyrique, l'acide valérique, des sels de ceux-ci, des esters de ceux-ci et des mélanges de ceux-ci.

5. Utilisation cosmétique selon l'une quelconque des revendications précédentes, dans laquelle l'au moins un acide gras à chaîne courte est obtenu à partir d'au moins un micro-organisme de la souche *Propionibacterium acnes* ATCC 6919.

6. Utilisation cosmétique selon la revendication 1, dans laquelle ledit milieu de culture conditionné est obtenu par le procédé comprenant les étapes suivantes :
i) culture d'au moins un micro-organisme de l'espèce *Propionibacterium acnes* telle que *Propionibacterium acnes* ATCC 6919 ;
ii) séparation, en particulier par centrifugation, du surnageant de culture de la biomasse ;
iii) récupération du surnageant de culture ; et
iv) stabilisation facultative du surnageant de culture, par exemple par filtration.

7. Utilisation cosmétique d'un milieu de culture conditionné tel que défini dans l'une quelconque des revendications 1 à 6, dans laquelle ledit milieu de culture conditionné est utilisé en combinaison avec au moins un acide gras à chaîne longue comprenant au moins 10 atomes de carbone, des esters non glycéryliques de ceux-ci et des mélanges de ceux-ci.

8. Utilisation cosmétique selon l'une quelconque des revendications précédentes, dans laquelle la peau sèche est **caractérisée par** la présentation d'une sensation d'inconfort telle que des tiraillements et/ou des tensions, un toucher rugueux et/ou des squames.

9. Méthode cosmétique non thérapeutique de prévention et/ou de traitement de la peau sèche et/ou de la peau âgée, en particulier de la peau sèche et/ou de la peau âgée hyposéborrhéique, comprenant l'application d'une composition cosmétique sur la peau, dans laquelle ladite composition comprend un milieu de culture conditionné tel que défini dans l'une quelconque des revendications 1 à 8.
